(19) **Europäisches Patentamt / European Patent Office / Office européen des brevets**

(11) **EP 3 210 574 A1**

(12) # EUROPEAN PATENT APPLICATION

(43) Date of publication:
**30.08.2017 Bulletin 2017/35**

(51) Int Cl.:
**A61F 2/16** (2006.01)

(21) Application number: **17382101.8**

(22) Date of filing: **28.02.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.02.2016 ES 201630234**

(71) Applicant: **Universidad De Murcia**
**30003 Murcia (ES)**

(72) Inventors:
- **FERNÁNDEZ MARTÍNEZ, Enrique Josua**
  **30003 Murcia (ES)**
- **ARTAL SORIANO, Pablo**
  **30003 Murcia (ES)**

(74) Representative: **Elzaburu S.L.P.**
**C/ Miguel Angel, 21**
**28010 Madrid (ES)**

(54) **INTRAOCULAR ABERRATION CORRECTION LENS**

(57) The intraocular aberration correction lens is shaped by an optical area that has a gradient in the chromatic dispersion value of the material or materials that shape it, in such way that said gradient is parallel to the optical axis. The net value of the chromatic dispersion in the anterior area of the lens is different from the value in its posterior area. For this, the use of a single material or various is possible. The anterior and posterior surfaces of the lens, as well as the separation between adjacent materials, if applicable, have a geometric shape so that the group comprising the intraocular lens and the eye that contains it display a correction, or significant reduction, of the optical aberrations, both the chromatic ones and the monochromatic ones on and outside the optical axis. The lens can be given areas with different optical powers in a way that enables clear and simultaneous vision at different distances.

FIG. 1

## Description

## Field of the invention

The present invention pertains to the group of intraocular aberration correction lens. The intervention refers to an intraocular lens that presents a calculated optical power in such a way that it provides an emmetropic refractive state.

The lens can replace the natural crystalline, either because of loss of transparency, the capacity to adjust its power, or to correct the refractive state of the eye, to enable correct vision without other optical supports. The lens can also be implanted while maintaining the patient's crystalline, in which case it is only used to correct the refractive state of the eye.

Therefore, the present intervention pertains to the field of medical prostheses that are implanted inside the eye.

## Prior art of the invention

### ABERRATIONS

In general, a chromatic aberration is understood to be the deviation of an optical system in relation to its ideal behaviour as the consequence of the change in the index, or indexes, of refraction, depending on the colour, frequency or wavelength of the light traversing it. For studying it, the chromatic aberration is generally divided into two, with attention to the object that produces them. Therefore, the longitudinal aberration is established for momentary objects and on the eye, as a difference in the focal plane depending on the wavelength of the light used. For momentary objects outside the eye, or extended objects, a transversal, lateral or increased chromatic aberration is used. This produces a change in the position or size of the images, depending on wavelength used. All means, except a vacuum, present change in its reflection index to a greater or lessor extent, depending on the wavelength used. This phenomenon is called chromatic dispersion. Different parametrized equations are usually used for its characterization, such as, for example, the Cauchy or Sellmeier equation. Using the Abbe number as a simplified way is also commonplace, with a single value for indicating if a material is more or less dispersive. The Abbe number is mathematically defined as:

$$V_d = \frac{n_d - 1}{n_F - n_C},$$

where $n_d$, $n_F$ y $n_C$ are the refraction indexes of the material in the wavelengths corresponding to the so called Fraunhofer lines d, F and C, that numerically correspond to 587.6 nm, 486.1 nm and 656.3 nm, respectively. Throughout this document, when it is indicated that one material is more or less dispersive than another, it must be understood in the sense of exhibiting a smaller or greater Abbe number, respectively, than the one taken as the comparison.

The eye, through the different optical means comprising it, such as the aqueous humour, vitreous humour, etc., presents a notable chromatic dispersion. Therefore, the images it produces on the retina are affected by a significant amount of chromatic aberration. As such, short wavelengths, which correspond to blue tones in the visible spectrum, are focused in front of those long wavelengths corresponding to reddish tones in the visible spectrum.

On the other hand, the aberrations called monochromatic aberrations are the ones that appear without the need of considering the chromatic dispersion of the mean or means that comprise the optic system, in a deviation shape with respect to the ideal or paraxial behaviour of the system. For study purposes, the ones usually classified as point or centre aberrations are the ones produced by momentary objects on the optical axis, and extra-axial or outside the axis aberrations, that arise as a consequence of the consideration of momentary objects outside the optic axis, or objects or extended scenes that cover a certain field in the subject space. Specifically, these are called field aberrations.

The human eye, understood as an image forming system, suffers from aberrations inside and outside the optic axis, as well as the previously mentioned chromatic aberrations that limit the optic quality of the images projected on the retina. With respect of the ideal behaviour, these deviations have an important relevance in the visual quality, with special impact on the peripheral vision.

### INTRAOCULAR LENS

At present, surgical implantation of intraocular lens, to replace the natural crystalline after a cataract operation, and as an ametropias corrector element like myopia or astigmatism in the anterior chamber of the eye, constitutes a routine practice. As detailed in the work of D.J. Apple and D. Sims, "Harold Ridley and the invention of the intraocular lens," Survey of Ophthalmology 40, 279-292 (1996), the technique was introduced by Ridley in 1945, based on the experience he acquired treating traumas military pilots suffered as a consequence of combat in the World War Two. Intraocular lens in their most extended form are designed to correct a patient's farsightedness. These are called monofocal lenses, because they are made with a single optical power. There are different formulas for obtaining the adequate power to correct the patient's farsightedness. These formulas require first measuring some biometric parameters of the eye, such as axial length, length of the anterior chamber, or an estimate of the position of the implanted lens. Some include the refraction of the object displays. The majority of people with implanted intraocular lens do not need additional corrections with glasses or contact lenses to correctly see far. Ideally, an intraocular lens should provide clear vision at every distance, just as the natural crystalline does for the large part of a lifetime. When the eye does not accommodate, it is called presbyopic. This capacity to accommodate is lost with age, and so the inclusion of this multifocality characteristic in the intraocular lens is of capital importance. In effect, the capacity to focus clearly on scenes at different distances is basic, and gives people quality of life and well-being. It is estimated that approximately 80% of the information a human being handles in industrialised societies is obtained by sight. This also occurs with short-sightedness, and mid-sightedness.

There are numerous alternatives for manufacturing intraocular lens. The following are some patents related to the present invention:

US 7 241 009 B2, US 7 837 326 B2, US 2011/0071 628 A1, US2002/0107 568 A1 and US 2006/0271 187A1 , US 2004/0015 236 A1, US2010/0211 171 A1, US 2011/0035001 A1, US 7 871 437 B2, US 6 217 619 B1, US 2003/0187 505 A1, US 2004/0249 456 A1, US 2005/0125058 A1 and US 2005/0107875 A1, US 2008/0154 363 A1, US 6 638 306 B2, US 2007/0129 801 A1, 2005/0107 875 A1, US 2008/0027 538 A1,US 2009/0234 449 A1, US 2010/0228 346 A1, US 7217 288 B2, US 7261 737 B2, US 7220 279 B2, US 5 158 572 A, US 2010/0100177A1, US 005 112 351 A, US 007 481 532 B2, US 2009/0240 328 A1, US 20 100 097 569 A1.

EP0329981A1, EP1862147A1, US5201762, US6391230, US20110037184A1,

US20140180409, US20070004863, US5152787, US6695880, US20120310340,

Many intraocular lens and optical elements of the prior art use a gradient in their refraction index, and incorporate variations in their values, as a function of the distance to the optical axis.

## Summary of the invention

The subject matter of the invention is to provide an aberration correction intraocular lens that can provide quality and clear vision free of chromatic and monochromatic aberrations within a wide field of view, improved in comparison with the vision provided by the intraocular lenses of the prior art.

The invention provides an intraocular aberration correction lens that comprises mechanical fasteners for implanting the lens in the interior of the eye, in which its optical area comprises at least a material that makes said optical area present a gradient in the chromatic dispersion in the direction of the optical axis, the effective value of the chromatic dispersion being different in the anterior area of the lens, which is the area most proximate to the cornea of the eye after the lens implantation, from that in the area posterior to the lens, which is the area most proximate to the retina of the eye after the lens is implanted.

The invention, therefore, consists in an intraocular lens made of a material or materials that present a gradient in its chromatic dispersion, in contrast to or as a novelty with respect of lenses and optical elements that use a gradient in their refraction index. Furthermore, this gradient can only be introduced in a direction that coincides with the optical axis of the eye; also as a novelty and advantage compared to lenses with an index gradient that incorporates variations in their values as a function of the distance to the optical axis.

The subject intraocular lens of the invention also has the possibility incorporated of correcting or manipulating the monochromatic aberrations with adequate carving of the available surfaces. The flexibility in the manufacturing of this new intraocular lens enables not only correcting or significantly reducing, or ultimately manipulating, the usual aberrations on the optical axis of the eye, but also the eccentric aberrations outside the axis or field that are present in the eye and which, to date, have never been compensated or altered in a controlled manner. The simultaneous correction or manipulation of the chromatic and monochromatic aberrations provide a significant increase in the patient's visual quality, and notably increments the depth of field. The latter makes possible a clear vision of scenes placed at various distances. To this end, the incorporation of mulitfocal areas on this material support with chromatic dispersion gradient, involves an advance and a clear advantage over the current state of the art, given that it is possible to reduce the power addition needed in order to be able to see at a determined distance by the effect of increasing the depth of field.

This greatly contributes to an improved visual quality that can be quantified as an increase in visual acuity, in sensitivity to contrast in photopic conditions, high illumination like scotopics, or low illumination.

## Brief description of the drawings

The following will illustrate, but not limit, the subject matter of the present invention, making reference to the accompanying drawings, which are:

Figure 1 is a schematic display of the fundamental pieces that comprise the optical portion of the intraocular lens, according to a sagittal section of it. The optical axis is shown by discontinuous lines.

Figures 2A and 2B display in a scheme and with levels of grey the change in the chromatic dispersion of two possible materials for its use in the optical area of the intraocular lens. The light tones correspond to low chromatic dispersions, with respect of the dark tones, which represent high chromatic dispersions

Figure 3 displays the variable distribution of chromatic dispersion values in a material to be used in the construction of the optical area of the intraocular lens. The chromatic dispersion remains constant within a well delimited area in the space, and at the same time is different from the value of the chromatic dispersion in the adjacent areas. The light tones correspond to low chromatic dispersions, with respect of the dark tones, which represent high chromatic dispersions

Figures 4A and 4B display sagittal sections of the optical area of the intraocular lens, according to two possible alternatives with the use of materials with different chromatic dispersions.

Figures 5A, 5B, and 5C schematically display the distribution of different powers, according to a front plane, with which the optical area for the intraocular lens can be enabled for producing the correct far, mid, and near vision, together with the eye.

Figures 6A and 6B schematically display the distribution of different powers, according to a front plane, with which the optical area for the intraocular lens can be enabled for producing, together with the eye, the correct vision at different distances with the use of diffractive profiles only, or in combination with purely refractive zones.

Figure 7 displays a sagittal cut of the intraocular lens, with its fasteners in the interior of the eye after being implanted.

**Detailed description of the Invention**

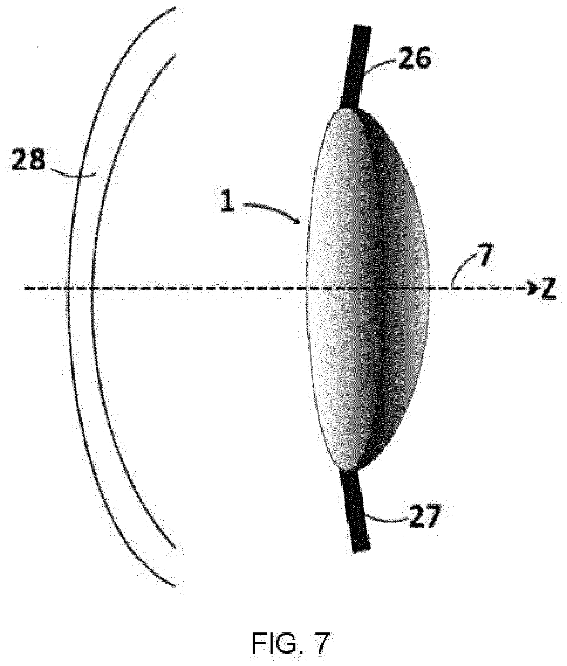

The present invention consists in a lens and its corresponding fastener 26, 27 (see Figure 7), designed for being implanted in human eyes. It's purpose is to provide quality vision that is clear and free of chromatic and monochromatic aberrations within a broad field of vision. It can be implanted in the human eye as a replacement of the natural crystalline, or in combination with it. In the first of the cases, it can be placed in a capsule, while in the second case it can be located in the anterior chamber. Its correct use enables clear vision without the need of other optical aids, such as glasses or contact lens in far vision, and eventually at other distances closer to the patient. To this end, a variant of the invention is proposed that incorporates areas with different powers for vision focussed on multiple distances.

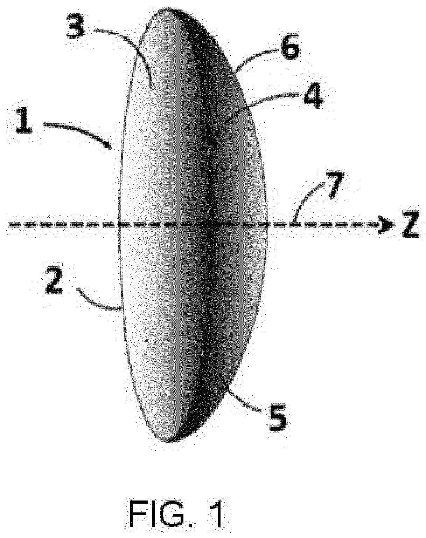

To attain the correction, or a significant decrease of the chromatic aberrations, optical area 1 of the intraocular lens, as displayed in Figure 1, is made up of one or several materials having the main characteristic of generating a net change in the values of the chromatic dispersion between the anterior area 3 of the lens and the posterior area 5. This is a novel concept, because there are precedents of lenses with refraction index gradient, but not with chromatic dispersion gradient. This is, however, the only variable parameter needed for attaining the correction of the chromatic aberration of the human eye. Following the usual notation, anterior area is understood to be the one closest to the cornea of the eye, whereas posterior area is understood to be the closest to the retina of the eye. Based on this description, it is possible to manufacture a family of intraocular lens with small variations in their parameters, which, however, obtain the same effect and are based on the same principle. To compensate the chromatic aberrations of the eye, the intraocular lens must introduce a chromatic aberration of equal magnitude and different sign than the one of the natural eye, so that the combination of the two produce the desired effect.

One of the simplest embodiments of the invention is one that only uses two materials with different chromatic dispersions, which would provoke the apparition of a separation or transition surface between them 4, o also with a single material with a continuous chromatic dispersion gradient. The combination of two materials, with each one showing a certain chromatic dispersion gradient, is also possible for the practical embodiment of the invention, as long as the net effect is a change between the values of the chromatic dispersion between the anterior and posterior areas. The minimum requirement, therefore, is that the chromatic dispersion gradient evolves along the optical axis, although variations in other directions are also acceptable.

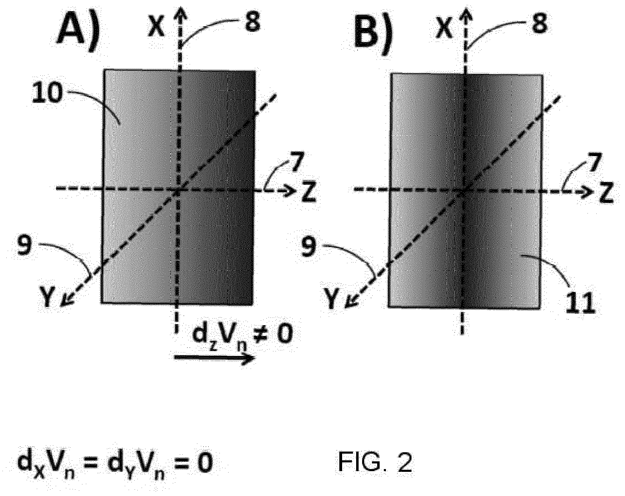

Figure 2 is a schematic display of an internal structure regarding the chromatic dispersion in an appropriate material for the intraocular lens. Part A) of Figure 2 displays a sagittal cut of the material where the different levels of grey indicate different chromatic dispersion values. White indicates low chromatic dispersion, while black corresponds to high chromatic dispersion. In principle, the value of the refraction index is not determinant for the invention, and the effect of the intraocular lens, depending on the latter, is the same after adjusting the other geometric parameters. This embodiment displays a material with a linear change and a monotonously increasing chromatic dispersion. Without losing the effect, other materials with an increasing chromatic dispersion could be used, without having to be linear, or even increasing in the whole interval. They must, however, ensure that the average value of the anterior and posterior areas of chromatic dispersion is significantly different. Figure 2 B) displays in a sagittal cut, another alternative for the material needed for the intraocular lens with a chromatic dispersion distribution that increases from the anterior portion up to a certain value of the optical axis Z 7, which in the case of Figure 2B, is made to coincide without loss of generality with the coordinates of the origin, and therefore with the X axis 8 and Y 9, from which it evolves with a decreasing linear tendency. Restrictions are not imposed on the functional shape of the evolution of the chromatic dispersion, which can be linear, quadratic, or whatever shape in general, as long as the net effect of the chromatic dispersion change is obtained between the anterior and posterior portions of the optical area of the intraocular lens. Further to the above, using a material that could be cut with an ample increasing chromatic dispersion would be possible, and also would have a clear benefit in the manufacturing of said lens, so that one piece would comprise the anterior area 3 of the lens, and perhaps with the other piece exhibiting a different thickness from the one used for the anterior area, and for the posterior area 5 of the lens, once it is glued with the orientation contrary to the initial piece. The minimum condition for the embodiment of the subject intraocular lens of this invention is expressed mathematically in Figure 2 by the equation $d_z V_n \neq 0$, which indicates that the differential or infinitesimal change of the Abbe $V_n$ number, coincident with the optical axis relative to the Z direction 7, is other than zero.

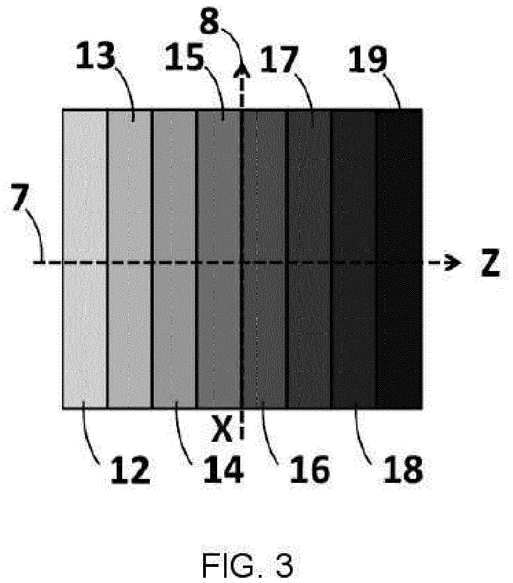

Another very interesting practical alternative for the internal distribution of the chromatic dispersion of the material that shapes the intraocular lens, is displayed schematically in Figure 3. In it, different layers of one or various materials whose main characteristic consists in the variation of the chromatic dispersion in differentiated areas, are placed sequentially along the length of axis Z 7. The main difference in regard to the embodiment displayed in Figures 2A and Figures 2B, consists in that the chromatic dispersion value now remains constant in a macroscopic spatial interval, for which it could provide a practical advantage by enabling the embodiment of the invention by gluing in any of its shapes on different materials. In said embodiment, the use of chemical glue is understood, by means of a product that enables the permanent bonding of the materials, like the mechanical glue. The latter can be carried out by means of a mechanical system which guarantees that the materials remain bonded due to mechanical forces and balances. Other gluing means not described here, but that allows the bonding of materials, are equally valid and produce the same effect. The use of a same material, with optical features that have been manipulated to obtain this described effect on the structure of the chromatic dispersion, is equally valid and does not involve a restriction for the embodiment of the present invention. It is even possible to obtain the same effect with materials that show a different chromatic dispersion and are not in mechanical contact, but rather separated by a given distance that can be controlled by using the fasteners designed for said purpose.

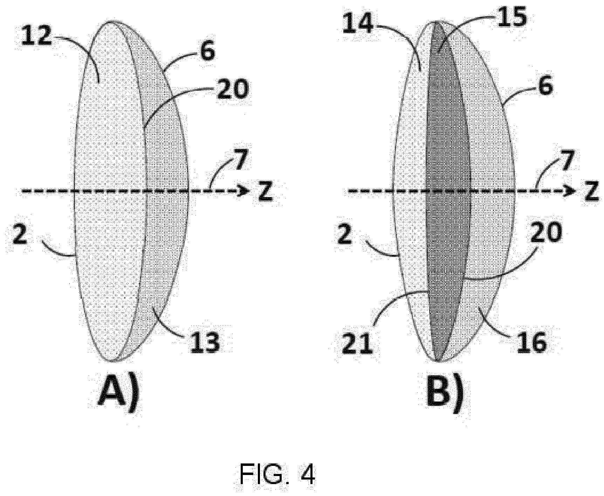

Figure 4 displays two preferred embodiments of the invention, which imply an advantage in their manufacture due to their simplicity. On the one hand, Figure 4A displays an implementation of the invention that only uses two materials having different chromatic dispersion, 12, 13, which generates three available surfaces, the anterior 2, posterior 6, and the internal separation surface between the two means 20. These surfaces 2, 20 and 6, are susceptible to being designed by means of specific geometric profiles for correcting aberrations in a wide field of the eye that contains the lens 1. A variation with an increasing complexity level is displayed in Figure 4B, with the conjunction of three materials 14, 15, 16, which generate an additional surface 21 on the previously displayed design in Figure 4A. The flexibility in the design is greater in this case, because one additional surface 21 is available for implementing the shapes needed for the simultaneous correction of the aberrations in the eye. An alternative to this embodiment of the invention consists in maintaining the materials that shape the intraocular lens without direct contact, which produces the same effect on the chromatic aberration, and also adds flexibility to the design. This is so because in the case of the lens displayed in Figure 4A, the condition in which the posterior limiting surface of the anterior means 12 may be equal to the anterior limiting surface of the posterior means 13, disappears. This fact facilitates the correction of the aberrations in the most simple way, making possible, for example, the complete correction of the spherochromatism, or dependence of the spherical aberration on the wavelength. Something similar occurs in the case of the lens in Figure 4B, when some of the means that shape 14, 15, 16, separate.

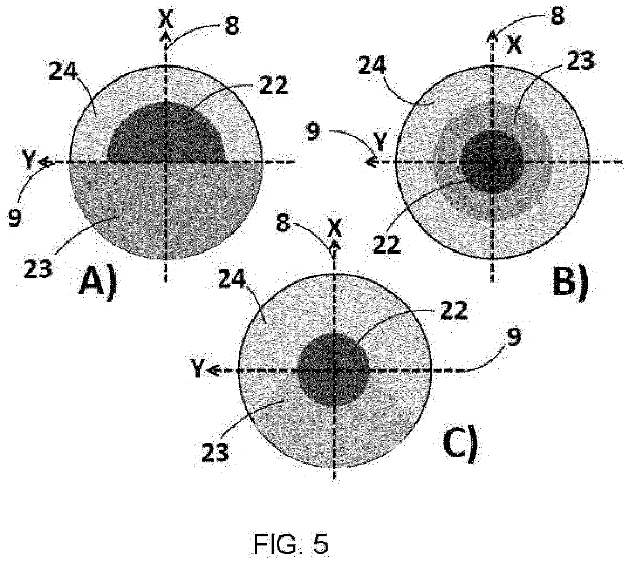

The subject intraocular lens of the present invention can be given multifocal capacity adding areas that display different optical power, as displayed in Figure 5. This figure displays different possible embodiments for incorporating said feature in the intraocular lens. Figure 5A displays a design that includes three differentiated areas, over plane X8 Y9, normal to optical axis 7 for far 24, intermediate 23, and near 22 sightedness. The distribution of areas dedicated to each distance is not limiting and can be varied without losing the effect. Moreover, it can be personalized for each patient, depending on his visual needs and lifestyle, and the number of areas and distances considered could even be increased or lowered. An equivalent but symmetric design around the optical axis 7, is displayed in Figure 5B, where concentric areas display different power for obtaining the effect of the multifocality. One big advantage of the present invention with respect of the state of the art, consists in the significant increase of the depth of field that the eye experiences from the effect of the correction of the chromatic and monochromatic aberrations. To this end, the areas dedicated to vision at different distances can be designed with lower power and the transition between them can be softer. This produces the immediate effect of an improvement of the optical quality in the multifocal versions with respect of others that do not use materials with chromatic dispersion gradient. Figure 5C displays another asymmetric alternative of the multifocal lens, in which the areas dedicated to each distance have been changed with respect of Figure 5A, in order to show the flexibility of the incorporation this property on the subject lens of the present invention. Another feature that represents an advance in regard to the flexibility and facility of the industrial production of the subject intraocular lens of this invention, compared to the ones that currently exist, consists in the possibility of distributing the necessary power change to obtain the multifocality between the different surfaces available on the lens. As such, in an embodiment like the one displayed in Figure 4B, it is possible to use the anterior surfaces 2, the posterior 6, and the separation between different materials 21 and 20 simultaneously, for distributing the power change, and then making the curvature changes in each area less abrupt than the ones needed for producing the same effect in a normal lens made with a single material.

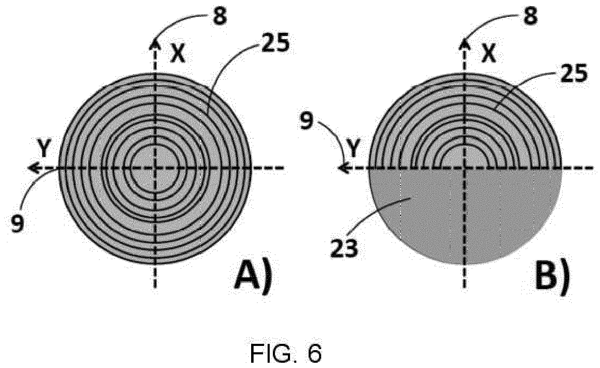

When producing areas that enable multifocality, one alternative is the sole use, or in combination, of the Fresnel profiles, also known as diffractive profiles. This is displayed schematically in Figure 6. Figure 6A is a schematic display of an idealization of the plane X 8 Y 9, perpendicular to the optical axis 7, one of the surfaces of the subject intraocular lens of this patent, in which the different areas with different optical power are obtained by carving a Fresnel or diffractive profile 25. Figure 6B graphically displays how use of optical power changes can be combined by combining a purely refractive area 23, with another diffractive one 25.

The subject intraocular lens of the present invention can be surgically placed in the anterior chamber of the eye or in the posterior chamber without loss of advantages, as shown in Figure 7. One or another position will determine the final parameters for its manufacturing, and the shape of the necessary fasteners 26, 27. These can be some of the ones that are currently are already used in the field, without changing the advantages and novelty that the intraocular lens described herein introduce.

The intraocular lens can be implanted to substitute the crystalline, or in conjunction with it, in what is known as a phakic lens. In both cases, the quality of the retinal images experience the advantages proper of the invention described herein.

In another embodiment of the intraocular lens of the invention, some of the materials that enable the generation of a chromatic dispersion gradient are separated from the rest of the materials that shape the lens and, therefore, surrounded by aqueous humour after the lens is implanted in the eye.

MATERIALS

Currently there is a broad range of possibilities regarding the materials that can be used in the intraocular lens. The current state of the art shows a large diversity of alternatives in the polymers family. Polymers, which consist in molecular chains whose fundamental unity is repeated in order to shape the structure of the material, constitute a preferred option. The macroscopic properties of the polymer, such as its refraction index, chromatic dispersion, water content, mechanical properties, etc., are determined by the properties of the so called base polymer molecule, as well as the way this molecule links to adjacent companions in the chain. The most used polymers in the ophthalmic optical field are the water-repellent and hydrophilic acrylics, and the silicones. Due to the nature of the intraocular lens disclosed herein and invented for use inside the eye of a patient, use of a biocompatible polymer, which has an inert behaviour once implanted inside the human body, is mandatory. There are numerous techniques that enable controlled variation of the chromatic dispersion of polymer materials, either during manufacturing or during the curing or maturing process prior to its carving or injection.

Technology enables manipulating the refraction indexes and the chromatic dispersions of the polymers for obtaining similar values starting from silicones or acrylic materials. Consequently, as regards materials the preferred embodiment of this invention does not need the detailed determination of the material type for its execution. Different alternatives give the same effect and benefit.

An alternative to using the usual polymers as regards the materials that shape the optical area of the intraocular lens, is the use of photopolymerizable materials. These open the possibility of being able to adjust its chromatic dispersion, refraction index, and even the geometrical shape, with controlled irradiation of ultraviolet light. The big advantage is that this polymerization can be carried out after implantation inside the eye of the patient. In this way there is a very high percentage of success in terms of the refraction obtained in the end. The use of this type of photopolymerizable material can be done in any of the materials of the lens, or several of them, 10, 11, 12, 13, 14, 15, 16 ,17, 18, 19, simultaneously. For this, the material closest to the anterior pole of the eye would have to be photopolymerized first, in order to progressively continue actuating on the rearmost materials. In any event, for the correct execution of this modality, the incorporation of an ultraviolet light filter is necessary for preventing the radiation used in the photopolymerization from reaching the retina of the subject.

The ultraviolet filter can be incorporated on the intraocular lens in any of the dioptres that comprise it, and there is no reason for its use to be linked to the use of photopolymerizable materials. In fact, its use is beneficial to the patient in every case, in as much it protects the cells of the retina of this portion of the spectrum, which is associated with various types of pathological degenerations.

There are numerous alternatives for the mechanical fasteners or haptics 26 and 27, that do not affect the properties and advantages of the present invention. As such, it can be used for the embodiment of the PMMA, polypropylene, polyamide, and vinylidene, poly fluoride or PVDF, or combinations thereof. All these previously disclosed materials are routinely used for manufacturing haptics in the field of intraocular lens.

A detailed description of the invention has been made with its preferred embodiments, as well as alternatives in many cases. However, there are other relatively evident modifications or variations that are obvious to an expert in the field of intraocular lens design, that have not been explicitly included. These other possible embodiments that are based on the same principles and ideas displayed in the present invention must also be understood as covered and protected by the present document.

The following numeric references are linked to the different elements described and represented in the present document:

1. Optical area of the intraocular aberration correction lens of the chromatic aberrations and aberrations outside the optical axis.

2. Anterior face of the intraocular aberration correction lens of the chromatic aberrations and aberrations outside the optical axis.

3. Anterior area of the intraocular lens with low chromatic dispersion material.

4. Separation surface between the anterior and posterior area of the intraocular lens.

5. Posterior area of the intraocular lens with high chromatic dispersion material.

6. Posterior face of the intraocular aberration correction lens of the chromatic aberrations and aberrations outside the optical axis.

7. Optical axis in the Z direction according to the usual Cartesian axes.

8. Axis perpendicular to the optical axis in the X direction according to the usual Cartesian axes.

9. Axis perpendicular to the optical axis in the Y direction according to the usual Cartesian axes.

10. Material with monotonously increasing variation of the chromatic dispersion according to the Z axis.

11. Material with monotonously increasing variation in the chromatic dispersion according to the Z axis in the anterior area, and monotonously decreasing in the posterior area.

12. Homogeneous and isotopic material with 13, 14, 15, 16, 17, 18, and 19, with a chromatic dispersion differing from the means and materials that surround it.

13. Homogeneous and isotopic material with 12, 14, 15, 16, 17, 18, and 19, with a chromatic dispersion differing from the means and materials that surround it.

14. Homogeneous and isotopic material with 12, 13, 15, 16, 17, 18, and 19, with a chromatic dispersion differing from the means and materials that surround it.

15. Homogeneous and isotopic material with 12, 13, 14, 16, 17, 18, and 19, with a chromatic dispersion differing from the means and materials that surround it.

16. Homogeneous and isotopic material with 12, 13, 14, 15, 17, 18, and 19, with a chromatic dispersion differing from the means and materials that surround it.

17. Homogeneous and isotopic material with 12, 13, 14, 15, 16, 18, and 19, with a chromatic dispersion differing from the means and materials that surround it.

18. Homogeneous and isotopic material with 12, 13, 14, 15, 16, 17, and 19, with a chromatic dispersion differing from the means and materials that surround it.

19. Homogeneous and isotopic material with 12, 13, 14, 15, 16, 17, and 18, with a chromatic dispersion differing from the means and materials that surround it.

20. Separation surface between two homogeneous and isotopic materials with different chromatic dispersions.

21. Separation surface between two homogeneous and isotopic materials with different chromatic dispersions.

22. Near-sighted area.

23. Mid-sighted area.

24. Far-sighted area.

25. Diffractive profile.

26. Fastener or haptic, fixed to 27 for the correct positioning of the lens in the interior of the eye.

27. Fastener or haptic, fixed to 26 for the correct positioning of the lens in the interior of the eye.

28. Cornea of the eye.

**Claims**

1. Intraocular aberration correction lens that comprises mechanical fasteners (26, 27), for its implanting in the lens inside the eye, **characterised in that** its optical area (1) comprises at least one material that makes said optical area (1) present a gradient in the chromatic dispersion in the direction of the optical axis (7) of the eye, the effective value of the chromatic dispersion being different in the anterior area (3) of the lens, which is the area most proximate to the cornea of the eye after the lens implantation, from that in the posterior area (5) of the lens, which is the area most proximate to the retina of the eye after the lens implantation.

2. Intraocular aberration correction lens according to claim 1, wherein its optical area (2) comprises a material (10, 11), with a continuous chromatic dispersion gradient.

3. Intraocular aberration correction lens according to claim 1, wherein its optical area (1) comprises two areas (3, 5), with materials having different chromatic dispersion gradient, among which there is a transition or separation surface (4).

4. Intraocular aberration correction lens according to claim 1, wherein its optical area (1) comprises two or more materials (12, 13, 14, 15, 16, 17, 18, 19) disposed in an aligned manner on the optical axis, in such a way that the chromatic dispersion between adjacent materials is different but remains constant in each one of said materials.

5. Intraocular aberration correction lens according to claim 4, wherein two or more materials (12, 13, 14, 15, 16, 17, 18, 19), are in contact with the anterior and/or with the following, having internal separation surfaces (20, 21).

6. Intraocular aberration correction lens, according to claim 4, wherein two or more materials (12, 13, 14, 15, 16, 17, 18, 19), are separated with respect of the anterior and/or with respect of the following at a proportional distance by means of fastening.

7. Intraocular aberration correction lens according to claim 5, wherein some of the anterior (2), posterior (6) or internal separation (20, 21) surfaces between materials (12, 13, 14, 15, 16, 17, 18, 19) present a geometric profile that corrects or significantly lessens the optical aberrations of the eye that contain it, including the ones of low order or spatial frequency like blur or astigmatism, and others of high frequency like spherical aberration and coma aberration, as well as chromatic aberration.

8. Intraocular aberration correction lens according to claim 5, wherein some of the anterior (2), posterior (6) or internal separation (20, 21) surfaces between materials (12, 13, 14, 15, 16, 17, 18, 19) present a geometric profile that acts on the optical aberrations of the group comprising the eye and the already implanted intraocular lens, increasing the depth of the field, and so enabling improved vision at different distances from the patient.

9. Intraocular aberration correction lens according to any of claims 5, 7, or 8, wherein some of the anterior (2), posterior (6) or internal separation (20, 21) surfaces between materials (12, 13, 14, 15, 16, 17, 18, 19) there are at least two areas that produce different optical power (22, 23, 24).

10. Intraocular aberration correction lens according to any of claims 5, 7, or 8, wherein in some of the anterior (2), posterior (6) or internal separation (20, 21) surfaces between materials (12, 13, 14, 15, 16, 17, 18, 19) there are two areas that produce different optical power (22, 23, 24) with Fresnel or diffractive lenses (25).

11. Intraocular aberration correction lens according to any of claims 3 to 10, wherein some of the materials that enable the generation of a chromatic dispersion gradient are separated from the rest of the materials that shape the lens and, therefore, surrounded by aqueous humour after the lens is implanted.

12. Intraocular aberration correction lens according to any of the previous claims, wherein the lens is made of a biocompatible polymer.

13. Intraocular aberration correction lens according to claim 12, wherein the lens is made of acrylic polymers or silicones.

14. Intraocular aberration correction lens according to any of the previous claims 1 to 11, wherein the lens is made of photopolymerizable materials and incorporate an ultraviolet light filter.

15. Intraocular aberration correction lens according to any of the previous claims, wherein the mechanical fasteners (26, 27) can be made of PMMA, polypropylene, polyamide, vinylidene poly fluoride, or combinations thereof.

FIG. 1

A)

B)

$d_x V_n = d_Y V_n = 0$

FIG. 2

FIG. 3

FIG. 4

FIG. 5

FIG. 6

FIG. 7

Europäisches
Patentamt

European
Patent Office

Office européen
des brevets

# EUROPEAN SEARCH REPORT

**Application Number**

EP 17 38 2101

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/168602 A1 (WEEBER HENDRIK A [NL]) 19 June 2014 (2014-06-19) | 1 | INV.<br>A61F2/16 |
| Y | * paragraph [0107] * | 2-15 | |
| Y | Arthur Bradley ET AL: "Acromatizing the Human Eye", Vol. 68, N°8, 1 January 1991 (1991-01-01), pages 608-616, XP055389749, Retrieved from the Internet: URL:http://pdfs.journals.lww.com/optvissci /1991/08000/Achromatizing_the_Human_Eye_.6 .pdf?token=method¦ExpireAbsolute;source¦Jo urnals;ttl¦1499773352044;payload¦mY8D3u1TC CsNvP5E421JYK6N6XICDamxByyYpaNzk7FKjTaa1Yz 22MivkHZqjGP4kdS2v0J76WGAnHACH69s21Csk0OpQ i3YbjEMdSoz2UhVybFqQxA71KwSU1A502zQZr96TQR whV1ocEp/s [retrieved on 2017-07-11] * page 610 * | 1 | |
| X,D | EP 1 862 147 A1 (ALCON INC [CH]) 5 December 2007 (2007-12-05) | 1 | TECHNICAL FIELDS SEARCHED (IPC)<br><br>A61F<br>G02C |
| Y | * paragraph [0036] - paragraph [0039] * | 2-15 | |
| X | EP 1 862 148 A1 (ALCON INC [CH]) 5 December 2007 (2007-12-05) | 1 | |
| Y | * paragraph [0040]; figures * | 2-15 | |
| Y,D | EP 0 329 981 A1 (ADVANCE MEDICAL S R L [IT]) 30 August 1989 (1989-08-30) * column 2, line 40 - column 4, line 3 * | 1-15 | |
| Y,D | US 2010/097569 A1 (WEEBER HENDRIK A [NL] ET AL) 22 April 2010 (2010-04-22) * the whole document * | 1-15 | |

The present search report has been drawn up for all claims

| Place of search | Date of completion of the search | Examiner |
|---|---|---|
| Munich | 12 July 2017 | Serra i Verdaguer, J |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**EP 3 210 574 A1**

## ANNEX TO THE EUROPEAN SEARCH REPORT
## ON EUROPEAN PATENT APPLICATION NO.

EP 17 38 2101

12-07-2017

| Patent document cited in search report | | | Publication date | Patent family member(s) | | | Publication date |
|---|---|---|---|---|---|---|---|
| US 2014168602 | A1 | | 19-06-2014 | AU | 2013308109 | A1 | 19-03-2015 |
| | | | | CA | 2883712 | A1 | 06-03-2014 |
| | | | | CN | 104755012 | A | 01-07-2015 |
| | | | | EP | 2890287 | A2 | 08-07-2015 |
| | | | | KR | 20150050588 | A | 08-05-2015 |
| | | | | US | 2014168602 | A1 | 19-06-2014 |
| | | | | US | 2016320633 | A1 | 03-11-2016 |
| | | | | WO | 2014033543 | A2 | 06-03-2014 |
| EP 1862147 | A1 | | 05-12-2007 | AR | 061416 | A1 | 27-08-2008 |
| | | | | AU | 2007202516 | A1 | 20-12-2007 |
| | | | | BR | PI0702544 | A | 19-02-2008 |
| | | | | CA | 2589601 | A1 | 30-11-2007 |
| | | | | CN | 101172056 | A | 07-05-2008 |
| | | | | EP | 1862147 | A1 | 05-12-2007 |
| | | | | JP | 2007319685 | A | 13-12-2007 |
| | | | | KR | 20070115740 | A | 06-12-2007 |
| | | | | RU | 2007120200 | A | 10-12-2008 |
| | | | | TW | 200806270 | A | 01-02-2008 |
| | | | | US | 2008147185 | A1 | 19-06-2008 |
| EP 1862148 | A1 | | 05-12-2007 | AR | 061417 | A1 | 27-08-2008 |
| | | | | AT | 444723 | T | 15-10-2009 |
| | | | | AU | 2007202517 | A1 | 20-12-2007 |
| | | | | BR | PI0702608 | A | 04-03-2008 |
| | | | | CA | 2589478 | A1 | 30-11-2007 |
| | | | | CN | 101181171 | A | 21-05-2008 |
| | | | | EP | 1862148 | A1 | 05-12-2007 |
| | | | | ES | 2332110 | T3 | 26-01-2010 |
| | | | | IL | 183368 | A | 29-12-2011 |
| | | | | JP | 2007319692 | A | 13-12-2007 |
| | | | | JP | 2013176607 | A | 09-09-2013 |
| | | | | KR | 20070115739 | A | 06-12-2007 |
| | | | | RU | 2007120244 | A | 10-12-2008 |
| | | | | TW | 200808278 | A | 16-02-2008 |
| | | | | US | 2007282438 | A1 | 06-12-2007 |
| EP 0329981 | A1 | | 30-08-1989 | EP | 0329981 | A1 | 30-08-1989 |
| | | | | IT | 1215851 | B | 22-02-1990 |
| US 2010097569 | A1 | | 22-04-2010 | AU | 2009306443 | A1 | 29-04-2010 |
| | | | | CA | 2741158 | A1 | 29-04-2010 |
| | | | | EP | 2364457 | A1 | 14-09-2011 |
| | | | | US | 2010097569 | A1 | 22-04-2010 |
| | | | | US | 2013090730 | A1 | 11-04-2013 |
| | | | | WO | 2010046356 | A1 | 29-04-2010 |

**ANNEX TO THE EUROPEAN SEARCH REPORT
ON EUROPEAN PATENT APPLICATION NO.** EP 17 38 2101

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2017

| Patent document cited in search report | Publication date | Patent family member(s) | Publication date |
|---|---|---|---|

For more details about this annex : see Official Journal of the European Patent Office, No. 12/82

EPO FORM P0459

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7241009 B2
- US 7837326 B2
- US 20110071628 A1
- US 20020107568 A1
- US 20060271187 A1
- US 20040015236 A1
- US 20100211171 A1
- US 20110035001 A1
- US 7871437 B2
- US 6217619 B1
- US 20030187505 A1
- US 20040249456 A1
- US 20050125058 A1
- US 20050107875 A1
- US 20080154363 A1
- US 6638306 B2
- US 20070129801 A1
- US 20080027538 A1
- US 20090234449 A1
- US 20100228346 A1
- US 7217288 B2
- US 7261737 B2
- US 7220279 B2
- US 5158572 A
- US 20100100177 A1
- US 005112351 A
- US 007481532 B2
- US 20090240328 A1
- US 20100097569 A1
- EP 0329981 A1
- EP 1862147 A1
- US 5201762 A
- US 6391230 B
- US 20110037184 A1
- US 20140180409 A
- US 20070004863 A
- US 5152787 A
- US 6695880 B
- US 20120310340 A

**Non-patent literature cited in the description**

- **D.J. APPLE ; D. SIMS.** Harold Ridley and the invention of the intraocular lens. *Survey of Ophthalmology,* 1996, vol. 40, 279-292